# EUROPEAN PATENT APPLICATION

(11) **EP 1 952 790 A1**
(43) Date of publication of application: **06.08.2008**
(21) Application number: 08100874.0
(22) Date of filing: 24.01.2008
(51) Int. Cl.: A61F 9/007

(54) **Membrane scraper**

(30) Priority: 30.01.2007 US 668607
(71) Applicant: Alcon, Inc., 6331 Hünenberg (CH)
(72) Inventor: Attinger, Jürg, 8260 Stein am Rhein (CH)
(74) Representative: Hanna, Peter William Derek

(57) **Abstract**

A membrane scraper (10,110) comprises a probe having a handle (12,112) and attached cannula (16,116). The cannula contains a snare or loop (18) of metal or polymer wire that can be either of fixed length or adjustable/retractable. At least a portion of the loop is roughened, either by roughening the loop material itself, or by coating the loop with an abrasive material, such as diamond dust or similar abrasive material. The membrane scraper affords greater visibility to the surgeon in ophthalmic surgery.

## Description

This invention relates to ophthalmic surgical equipment and more particularly to posterior segment ophthalmic surgical equipment.

### Background of the Invention

A continuing challenge to vitreoretinal surgeons is the separation of proliferative membranes from the neurosensory retina without injury or harm to the neurosensory retina during treatment for proliferative vitreoretinal disorders. For such a treatment, the removal of proliferative membranes from the surface of the retina is required in a wide variety of pathologic conditions and surgical situations. Various intraocular picks and intraocular forceps have been previously used for the removal of proliferative membranes.

However, prior art proliferative membrane removal techniques using conventional instruments carries the risk of causing damage to the retina at all times. In addition, "immature proliferative membranes", seen in some proliferative vitreoretinal disorders, may be friable, difficult to peel off as films, and often cannot be sufficiently removed from the surface of the retina. The unremoved or remaining proliferative membranes can be the source of subsequent reproliferation over time.

One prior art device, disclosed in U.S. Patent No. 5,921,998 (Tano, et al.) and commercially marketed as the "Tano Scraper" consists of a rigid cannula having a relatively flexible tube attached to its distal end. The tube is coated with an abrasive, such as diamond dust. The distal end of the cannula is rubbed over the tissue to be removed, providing a gentle scrubbing or sanding action. As the flexible tube is opaque, it can be difficult to see the surface being rubbed.

Accordingly, a need continues to exist for a membrane scraper having increased visibility.

### Brief Summary of the Invention

The present invention improves upon prior art by providing a membrane scraper in accordance with claims which follow. The membrane scraper comprises a handle and attached cannula. The cannula contains a snare or loop of metal or polymer wire that can be either of fixed length or adjustable/retractable. At least a portion of the loop is roughened, either by roughening the loop material itself, or by coating the loop with an abrasive material, such as diamond dust or similar abrasive material.

Accordingly, an objective of the present invention to provide an ophthalmic membrane scraper.

Another objective of the present invention is to provide a membrane scraper having increases visibility of the surface being scraped.

Another objective of the present invention to provide an ophthalmic membrane scraper having a loop or snare.

A further objective of the present invention to provide an ophthalmic membrane scraper having a roughened loop or snare.

Other objectives, features and advantages of the present invention will become apparent with reference to the drawings, and the following description of the drawings and claims.

### Brief Description of the Drawings

FIG. 1 is an enlarged perspective view of a first embodiment of the membrane scraper of the present invention.
FIG. 2 is an enlarged perspective view of one embodiment of the distal tip of the membrane scraper of the present invention, taken at circle 2 in FIG. 1.
FIG. 3 is an enlarged cross-sectional view of a second embodiment of the membrane scraper of the present invention.
FIG. 4 is an enlarged perspective view of a second embodiment of the distal tip of the membrane scraper of the present invention, taken at circle 4 in FIG. 3.

### Detailed Description of the Drawings

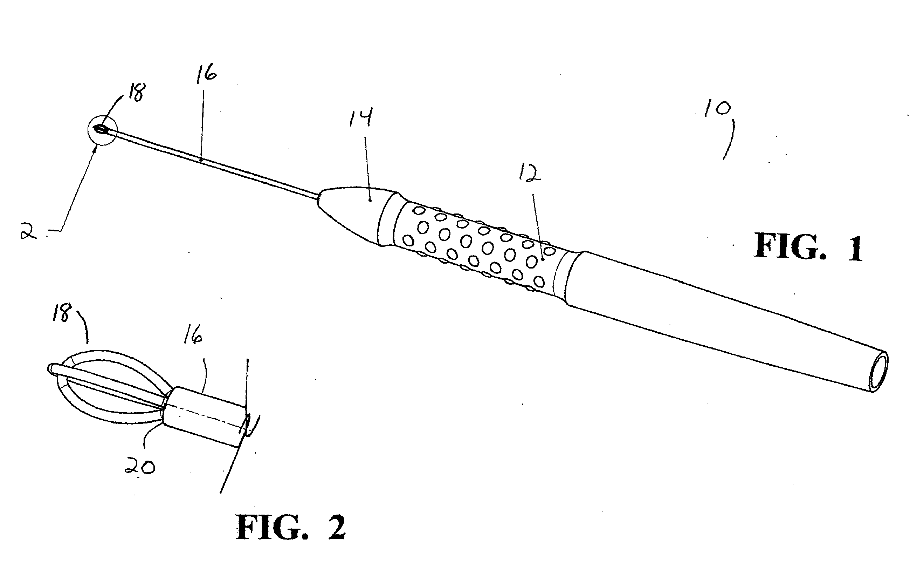

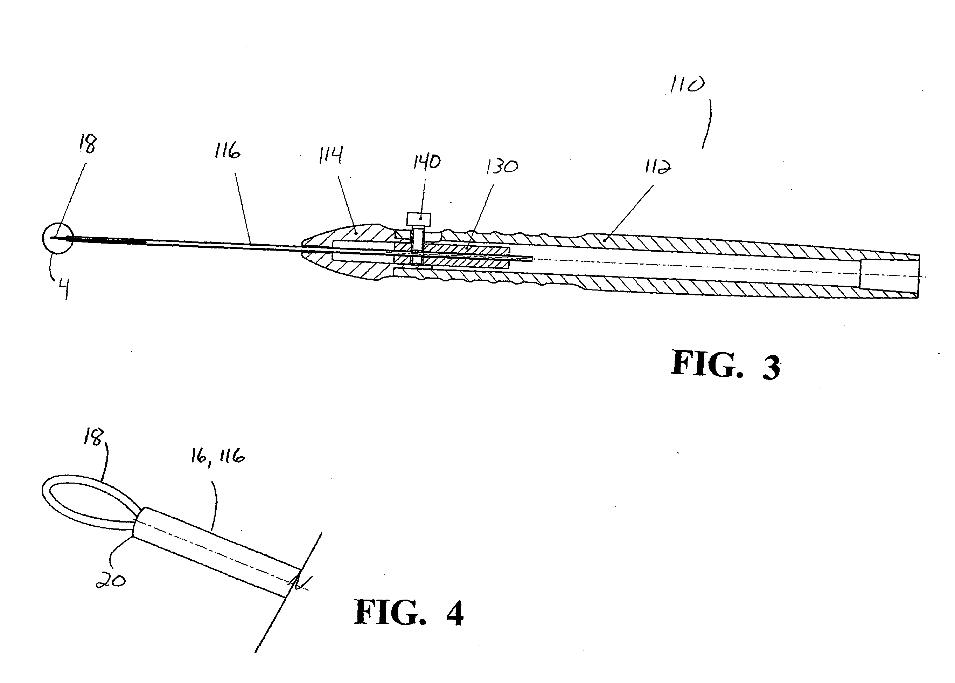

As best seen in FIG. 1, a first embodiment of membrane scraper 10 of the present invention generally includes handle 12, nosecone or hub 14, cannula 16 and loop or snare 18. Cannula 16 attaches to hub 14 and hub 14 attaches to handle 12 by any of a variety of know attachments methods, such as adhesive, ultrasonic welding, heat welding or crimping. Cannula 16 preferably is made from 20 ga., 23 ga. or 25 ga. or smaller gauge stainless steel or titanium tubing, and hub 14 and handle 12 preferably is molded from thermoplastic or formed from stainless steel or titanium by methods well-known in the art. Loop 18 preferably is formed from stainless steel, titanium or polymer wire and attached to distal end 20 of cannula 16 by adhesive, welding or crimping. As best seen in FIGS. 2 and 4, membrane scraper 10 may contain a single loop 18 or a plurality of loops 18. Loop(s) 18 preferably have a roughened texture, which can be accomplished by roughening loop 18 directly (*e*.*g*., sandblasting or cutting serrations into loop 18) or by applying a roughening coating to loop 18 (*e*.*g*., diamond or other mineral or metallic dust). Loop(s) 18 allow the surface being acted upon to be visualized more easily, which is especially important with delicate eye tissues.

Alternatively, as best seen in FIG. 3, membrane scraper 110 may contain loop(s) 18 that extend through cannula 116 and be attached to sliding block 130 that reciprocates within hollow handle 112 and nosecone 114. Reciprocation of block 130 is achieved by manipulation of button or lever 140. Movement of button 140 forwards (distally) causes loop(s) 18 to be pushed forward and extend out of distal end 20 of cannula 116, as shown in FIGS. 2 and 4. Movement of button 140 rearwards (proximally) causes loop(s) 18 to be retracted within distal end 20 of cannula 116.

While certain embodiments of the present invention have been described above, these descriptions are given for purposes of illustration and explanation. Variations, changes, modifications and departures from the systems and methods disclosed above may be adopted without departure from the scope of the present invention.

## Claims

1. A membrane scraper (10,110), comprising a rigid cannula (16,116) having a relatively flexible and abrasive scraper member attached to its distal end (20),
**characterized in that**
the scraper member comprises at least one wire loop or snare (18).

2. The membrane scraper of claim 1, further comprising a handle (12,112) having a nosecone (14,114), wherein the cannula (16,116) is attached to the nosecone.

3. The membrane scraper of claim 2, wherein the handle (112) is hollow and comprises a sliding block (130), the at least one wire loop or snare (18) extending through the cannula (116) and being attached to the sliding block, such that movement of the sliding block causes the at least one wire loop or snare to reciprocate within the cannula.

4. The membrane scraper of any one of claims 1 to 3, comprising a plurality of wire loops or snares (18).

5. The membrane scraper of any one of claims 1 to 4, wherein the wire loop(s) or snare(s) (18) is/are roughened.

6. The membrane scraper of any one of claims 1 to 4, wherein the wire loop(s) or snare(s) (18) comprise(s) a roughening coating.
